(19) Europäisches Patentamt
European Patent Office
Office européen des brevets

(11) Publication number: 0 159 565 A1

# (12) EUROPEAN PATENT APPLICATION

(21) Application number: 85103688.9

(22) Date of filing: 28.03.85

(51) Int. Cl.⁴: C 07 D 307/80, A 61 K 31/34 // C07C43/23, C07C39/19

(30) Priority: 02.04.84 US 596139

(43) Date of publication of application: 30.10.85 Bulletin 85/44

(84) Designated Contracting States: CH DE FR GB IT LI NL

(71) Applicant: MERCK & CO. INC., 126, East Lincoln Avenue P.O. Box 2000, Rahway New Jersey 07065 (US)

(72) Inventor: Chang, Michael N., 746 Austin Street, Westfield New Jersey 07090 (US)
Inventor: Hwang, San-Bao, 67 Canterbury Drive, Scotch Plains New Jersey 07076 (US)

(74) Representative: Abitz, Walter, Dr.-Ing. et al, Abitz, Morf, Gritschneder, Freiherr von Wittgenstein Postfach 86 01 09, D-8000 München 86 (DE)

## (54) Substituted 2,3,3alpha,6-tetrahydro-6-oxabenzofuran derivatives useful as paf antagonists.

(57) Substituted 2,3,3α,6-tetrahydro-6-oxabenzofuran derivatives of formula:

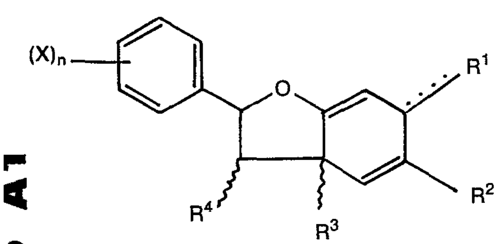

(I)

have been prepared wherein
$R^1$ is (1) when it is joined to the ring by a double bond, e.g., O or S; or
(2) when it is joined to the ring by a single bond, e.g., $SR^5$ where $R^5$ represents, e.g., H or loweralkyl;
$R^2$ is, eg., loweralkyl;
$R^3$ is, e.g., $OR^5$ where $R^5$ is as previously defined except that it cannot be $CH_3$;
$R^4$ is, e.g., loweralkyl;
X is, e.g., H or loweralkyl; and
n is, e.g., 2.

These neolignan compounds are found to have potent and specific PAF (Platelet-Activating-Factor) antagonistic activities and thereby useful in the treatment of various diseases or disorders mediated by the PAF, for example, pain, fever, inflammation, cardiovascular disorder, asthma, lung edema, allergic disorders, skin diseases, psoriasis, and adult respiratory distress syndrome.

EP 0 159 565 A1

TITLE OF THE INVENTION:

SUBSTITUTED 2,3,3α,6-TETRAHYDRO-6-OXABENZOFURAN DERIVATIVES USEFUL AS PAF ANTAGONISTS

BACKGROUND OF THE INVENTION

Platelet-activating factor (PAF) has recently been identified as an acetyl glyceryl ether phosphorylcholine (AGEPC), i.e., 1-O-hexadecyl/octadecyl-2-O-acetyl-sn-glyceryl-3-phosphorylcholine (Hanahan, D. S. et al., J. Biol. Chem., 255:5514, 1980). Even before its chemical identification, PAF has been linked to various biologic activities and pathways making it one of the important mediators responsible for a variety of physiological process including activation or coagulation of platelets, pathogenesis of immune complex deposition, smooth muscle contraction, inflammation as well as respiratory, cardiovascular and intravascular alterations. These physiological processes are known

to be associated with a large group of diseases, for example, inlfammatory diseases, cardiovascular disorders, asthma, lung edema, and adult respiratory distress syndrome. It is therefore only natural that more and more scientific investigators are focusing their work on the search of a PAF-antagonist or inhibitor for the treatment and/or the prevention of these common diseases.

The compounds of the present invention are potent and specific PAF-antagonists. They belong to the class of neolignan compounds related to piperenone, a known insect antifeeding substance, and 5-allyl-2-(3,4-dimethoxyphenyl)-3α,α-methoxy-3-methyl-2,3,3α,6-tetrahydro-6-oxobenzofuran, the subject matter of copending application Serial No. 541,806, filed October 13, 1983 (our Case 17003). Both piperenone and the 5-allyl compound mentioned above were isolated from the Chinese herbal plant Piper futokadzsura Sieb. See K. Matsui et al., Agr. Biol. Chem. 40, 1045 (1976); ibid, 40, 1113 (1976); and Matsui et al., Tetrahedron Letters 24, 1905 (1975). Although the plant has been used in Chinese herb medicine for the treatment of arthritis conditions, no one had successfully isolated nor identified the active substance until our work on the 5-allyl compound and the compounds of the present invention. We found these compounds are potent and specific PAF-antagonists useful not only in the treatment of arthritic conditions but also for other diseases including asthma, hypertension, lung-edema, adult distress syndrome and the like.

Accordingly, it is the object of the present invention to provide a class of novel compounds as specific PAF-antagonists.

Another object of this invention is to provide processes for the preparation of these PAF-antagonists.

A further object of this invention is to provide a pharmaceutically acceptable composition containing these novel compounds as the active ingredient for the treatment of diseases which are subject to the mediation of a PAF.

Still a further object of this invention is to provide a method of treatment comprising the administration of a therapeutically sufficient amount of one or more of the PAF-antagonists to a patient suffering from various skeletal-muscular disorders including but not limited to inflammation, e.g., osteoarthritis, rheumatoid arthritis and gout; allergic disorders; hypertension; cardiovascular disorder; asthma; lung edema; skin diseases; psoriasis; or adult respiratory distress syndrome.

DETAILED DESCRIPTION OF THE INVENTION

A. Scope of the Invention

This invention relates to the specific PAF-antagonists of formula:

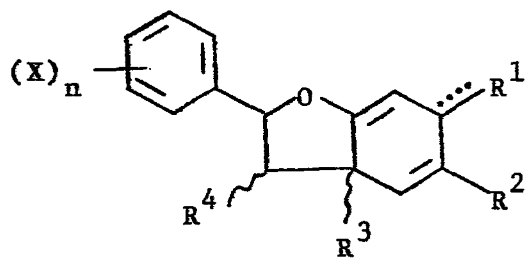

(I)

wherein

$R^1$ is (1) when it is joined to the ring by a double bond, O, S, $CR^5R^6$, or $NR^5$ where $R^5$ and $R^6$ independently represent H, loweralkyl, haloloweralkyl, loweralkenyl, aryl or aralkyl as defined below; or

(2) when it is joined to the ring by a single bond, $SR^5$, $SOR^5$, $SO_2R^5$, $NR^5R^6$, or $OR^7$ wherein $R^7$ represents $R^5$, $COR^5$, $COOR^5$, $CSR^5$ or $COSR^5$;

$R^2$ is (a) loweralkyl especially $C_{1-6}$alkyl such as methyl, n-propyl, i-propyl, t-butyl, cyclopropyl, n-hexyl, cyclopentyl or cyclohexyl;

(b) loweralkenyl especially $C_{1-6}$ alkenyl such as vinyl, allyl, $-CH_2CH{=}CH{-}CH_3$ or $-CH_2{-}CH_2CH{=}CHCH_2CH_3$; or

(c) loweralkoxy especially $C_{1-6}$ alkoxy such as methoxy, ethoxy or propoxy;

(d) lower alkynyl especially $C_{1-6}$ alkynyl, e.g. $-CH_2-C\equiv CH$ and $-CH_2-C\equiv C-CH_3$;

(e) aralkyl especially aryl loweralkyl such as benzyl or substituted benzyl, e.g., p-methoxybenzyl, m,p-dinitrobenzyl o,p-difluorobenzyl or p-methylbenzyl;

(f) haloloweralkyl especially $CF_3$;

$R^3$ is (a) loweralkyl especially $C_{1-6}$ alkyl;

(b) aryl especially phenyl or substituted phenyl such as p-methoxyphenyl, 2,4-dichlorophenyl, p-methylphenyl or the like; or

(c) loweralkenyl especially $C_{1-6}$ alkenyl;

(d) $OR^5$ where $R^5$ is as previously defined except that it cannot be $CH_3$;

(e) $SR^5$;

(f) $NR^5R^6$; or

(g) haloloweralkyl;

$R^4$ is (a) loweralkyl especially $C_{1-6}$ alkyl;

(b) lower alkenyl;

(c) lower alkynyl;

(d) hydrogen; or

(e) aralkyl;

X is (1) H;

(2) loweralkyl especially $C_{1-6}$ alkyl such as methyl, ethyl, propyl, t-butyl, pentyl, benzyl, cyclopropyl, cyclopentyl or cyclohexyl;

(3) loweralkenyl especially $C_{2-6}$ alkenyl, for example, vinyl, allyl, and buten-2-yl;

(4) loweralkynyl especially $C_{2-6}$ alkynyl such as $-C\equiv CH$, $-CH_2C\equiv CH$, $-C\equiv C-CH_3$;

(5) aryl of 6 to 10 carbons especially phenyl or substituted phenyl of formula $Ar(X')_n$ wherein X' independently is X except as defined in items (5) or (6), n is 1 to 5;

(6) aralkyl especially aryl loweralkyl such as benzyl or $Ar(X')_n$-loweralkyl;

(7) $R^5O-$;

(8) $R^5CO$;

(9) $R^5COO$;

(10) $R^5COS$;

(11) $R^5OCO$;

(12) $R^5SCO$;

(13) $R^5CONR^6$;

(14) $R^5NR^6CO$;

(15) $R^5R^6N$;

(16) $R^5S$;

(17) $R^5SO$;

(18) $R^5SO_2$;

(19) halo especially fluoro, chloro or bromo;

(20) $-O(CH_2)_mO-$ where m represents 1 or 2;

(21) $-(NH_2)C=NH$;

(22) -CN;

(23) $-NO_2$;

(24) $-(CH_2)_mOR^5$;
(25) $-(CH_2)_mCOOR^5$;
(26) heteroaryl or substituted heteroaryl of formula $Q_rX'$ where $Q_r$ is heteroaryl such as thienyl, furyl, pyridyl, pyrazinyl, pyrimidinyl, pyridazinyl, triazinyl, isoxazolyl, isothiazolyl pyrryl, imidazolyl, pyrazolyl, pyranyl, 2H-pyrryl, imidazolidinyl, imidazolinyl, pyrazolidinyl, pyrrolidinyl, pyrrolinyl, piperidyl, piperazinyl, morpholinyl, tetrazolyl, indolyl or the like; and X' is as previously defined; or
(27) halo loweralkyl especially $CF_3$-; and

n is 1 to 5.

Preferably, the antagonists of the present invention are of the structural formula (II).

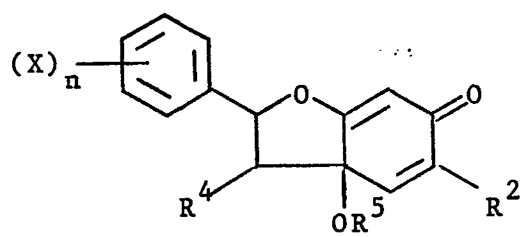

(II)

wherein $R^2$, $R^5$, $R^4$, X and n are as previously defined.

More preferably the antagonists of the present invention are of the structural formula (III)

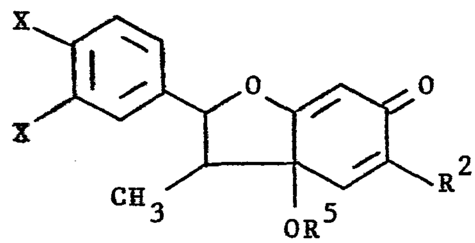

wherein $R^2$, $R^5$ and X are as previously defined.

The most preferred antagonists of this invention are those having the following conformation:

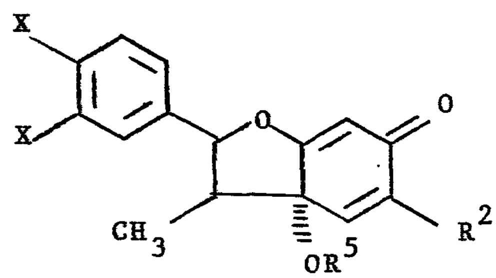

B. Preparation of the compounds within the scope of the invention

The novel compounds of the present invention can be prepared by the following representative process:

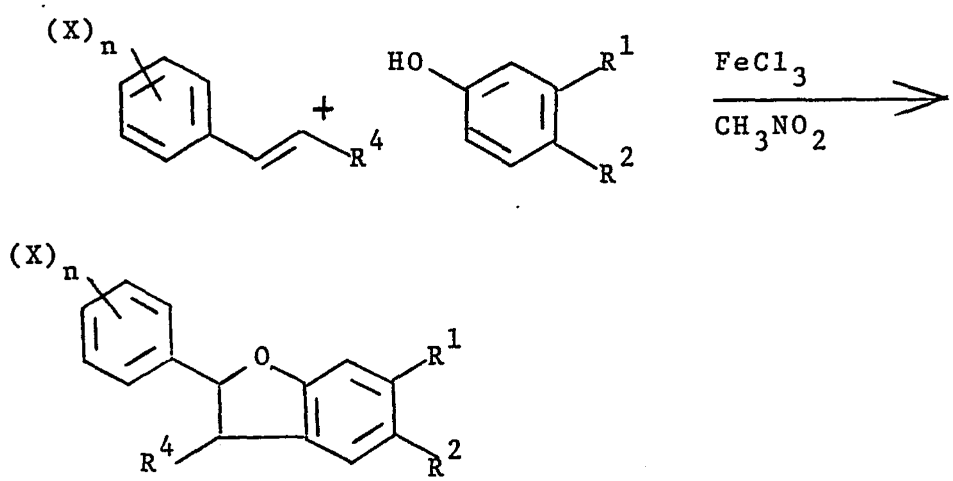


(III)

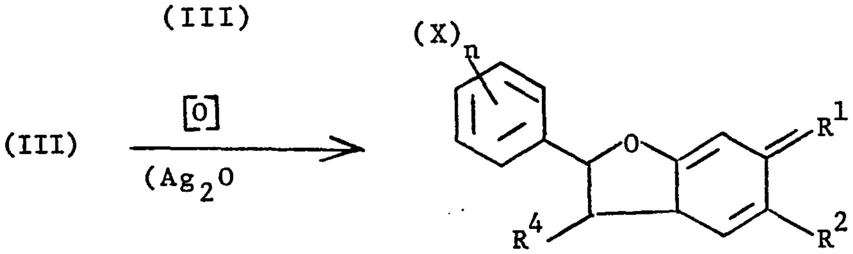


(IV)

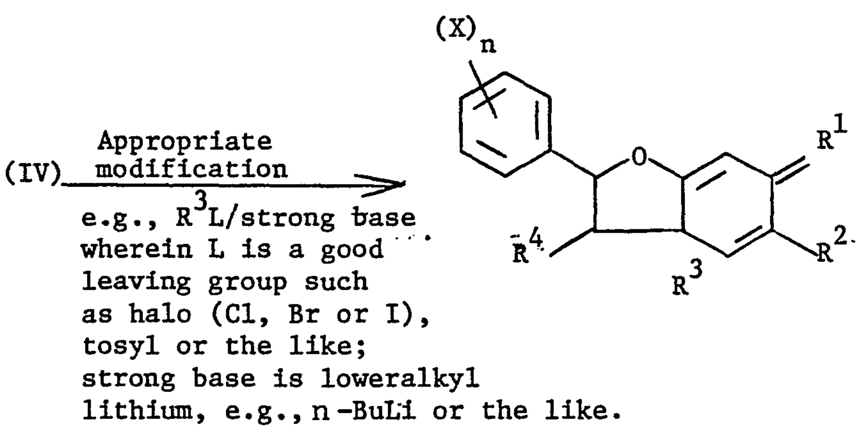


(I)

C. Utility of the compounds within the scope of the invention

This invention also relates to a method of treatment for patients (or mammalian animals rasied in the diary, meat, or fur industries or as pets) suffering from disorders or diseases which can be attributed to PAF as previously described, and more specifically, a method of treatment involving the administration of a compound of formula (I) as the active constituent.

Accordingly, the compounds of formula (I) can be used among other things to reduce inflammation, to correct respiratory, cardiovascular, and intravascular alterations or disorders, and to regulate the activation or coagulation of platelets, the pathogenesis of immune complex deposition and smooth muscle contractions.

For the treatment of inflammation, cardiovascular disorder, asthma, or other diseases mediated by the PAF, a compound of formula (I) may be administered orally, topically, parenterally, by inhalation spray or rectally in dosage unit formulations containing conventional non-toxic pharmaceutically acceptable carriers, adjuvants and vehicles. The term parenteral as used herein includes subcutaneous injections, intravenous, intramuscular, intrasternal injection or infusion techniques. In addition to the treatment of warm-blooded animals such as horses, cattle, sheep, dogs, cats, etc., the compounds of the invention are effective in the treatment of humans.

The pharmaceutical compositions containing the active ingredient may be in a form suitable for oral use, for example, as tablets, troches, lozenges, aqueous or oily suspensions, dispersible powders or granules, emulsions, hard or soft capsules, or syrups or elixirs. Compositions intended for oral use may be prepared according to any method known to the art for the manufacture of pharmaceutical compositions and such compositions may contain one or more agents selected from the group consisting of sweetening agents, flavoring agents, coloring agents and preserving agents in order to provide pharmaceutically elegant and palatable preparation. Tablets contain the active ingredient in admixture with non-toxic pharmaceutically acceptable excipients which are suitable for the manufacture of tablets. These excipients may be for example, inert diluents, such as calcium carbonate, sodium carbonate, lactose, calcium phosphate or sodium phosphate; granulating and disintegrating agents, for example, corn starch, or alginic acid; binding agents, for example starch, gelatin or acacia, and lubricating agents, for example magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monostearate or glyceryl distearate may be employed. They may also be coated by the techniques described in the U.S. Patents 4,256,108; 4,160,452; and 4,265,874 to form osmotic therapeutic tablets for controlled release.

Formulations for oral use may also be presented as hard gelatin capsules wherein the active ingredient is mixed with an inert solid diluent, for example, calcium carbonate, calcium phosphate or koalin, or as soft gelatin capsules wherein the active ingredient is mixed with water or an oil medium, for example peanut oil, liquid paraffin, or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropylmethylcellulose, sodium alginate, polyvinylpyrrolidone, gum tragacanth and gum acacia; dispersing or wetting agents may be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethyleneoxycetanol, or condensation products of ethylene oxide with partial esters derived from fatty acids and a hexitol such as polyoxethylene sorbitol monooleate, or condensation products of ethylene oxide with partial esters derived from fatty acids and hexitol anhydrides, for example polyxyethylene sorbitan monooleate. The aqueous suspensions may also contain one or more preservatives, for example ethyl, or n-propyl, p-hydroxybenzoate, one or more coloring agents, one or more flavoring agents, and one or more sweetening agents, such as sucrose or saccharin.

Oily suspension may be formulated by suspending the active ingredient in a vegetable oil, for example arachis oil, olive oil, sesame oil or coconut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide a palatable oral preparation. These compositions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, may also be present.

The pharmaceutical compositions of the invention may also be in the form of oil-in-water emulsions. The oily phase may be a vegetable oil, for example olive oil or arachis oils, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan mono-oleate, and

condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents. The pharmaceutical compositions may be in the form of a sterile injectable aqueous or oleagenous suspension. This suspension may be formulated according to the known art using those suitable dispersing or wetting agents and suspending agents which have been mentioned above. The sterile injectable preparation may also be a sterile injectable solution or suspension in a non-toxic parenterally-acceptable diluent or solvent, for example as a solution in 1,3-butane diol. Among the acceptable vehicles and solvents that may be employed are water, Ringer's solution and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

A compound of (I) may also be administered in the form of suppositories for rectal administration of the drug. These compositions can be prepared by mixing the drug with a suitable non-irritating excipient which is solid at ordinary temperatures but

liquid at the rectal temperature and will therefore melt in the rectum to release the drug. Such materials are cocoa butter and polyethylene glycols.

For topical use, creams, ointments, jellies, solutions or suspensions, etc., containing the anti-inflammatory agents are employed.

Dosage levels of the order from about 1 mg to about 100 mg per kilogram of body weight per day are useful in the treatment of the above-indicated conditions (from about 50 mg to about 5 gms. per patient per day). For example, inflammation is effectively treated and anti-pyretic and analgesic activity manifested by the administration from about 25 to about 75 mg of the compound per kilogram of body weight per day (about 75 mg to about 3.75 gms per patient per day). Advantageously, from about 5 mg to about 50 mg per kilogram of body weight per daily dosage produces highly effective results (about 250 mg to about 2.5 gm per patient per day).

The amount of active ingredient that may be combined with the carrier materials to produce a single dosage form will vary depending upon the host treated and the particular mode of administration. For example, a formulation intended for the oral administration of humans may contain from 5 mg to 5 gm of active agent compounded with an appropriate and convenient amount of carrier material which may vary from about 5 to about 95 percent of the total composition. Dosage unit forms will generally contain between from about 25 mg to about 500 mg of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The following examples serve to illustrate but are not intended to limit the scope of the present invention.

EXAMPLE 1

5-Allyl-2-(3,4-dimethoxyphenyl)-3,$3_{\alpha,\alpha}$-dimethyl-2,3,$3_{\alpha}$,6-tetrahydrobenzofuran

Step A: Preparation of 3-Propenoxy phenol

In a 250 ml round bottom flask, resorcinol (11.0 g, 0.1 mole) is dissolved in 100 ml THF and the solution was stirred at 0°C under nitrogen atmosphere. Sodium hydride (2.4 g, 0.1 mole, 97%) is added. After hydrogen evolution ceases, allyl bromide (12.3 g, 0.1 mole) is added slowly and the resulting mixture is heated to 45°C for 3 hours. The cooled reaction mixture is then poured into 200 ml ice water/200 ml ethyl acetate. The organic layer is dried with anhydrous sodium sulfate and concentrated to a yellow oil. This crude product of 3-propenoxy-phenol is used without further purification.

Step B: Preparation of 4-Propenyl resorcinol

A solution of 12.0 g (0.08 mole) of 3-propenoxy phenol in 50 ml toluene and trace p-toluene sulfonic peracid is heated to reflux for 2 hours. Toluene is distilled off and the remaining yellow oil is distilled at 0.1 mm and 125°C to yield a colorless oil (80%) of 4-propenyl resorcinol.

Step C: Preparation of 5-Allyl-2-(3,4-dimethoxyphenyl)-3-methyl-2,3-dihydro-6-oxabenzofuran

In a 250 ml round bottom flask, 4-propenyl resorcinol (5.0 g, 33 mmole) and 3,4-dimethoxyphenyl-2-propene (5.6 g, 33 mmole) are dissolved in 100 ml nitromethane, 100 mg of $FeCl_3$ is added and the resulting mixture is heated to 80°C for six hours. It is cooled to the room temperature and the solvent nitromethane is removed under reduced pressure. The residue is flash columned (Kiesel gel 60 silica, 20% ethyl acetate in hexane) and the crude product is recovered and crystallized from ethyl acetate/hexane to afford 5-allyl-2-(3,4-dimethoxyphenyl)-3-methyl-2,3-dihydro-6-oxabenzofuran.

Step D: Preparation of 5-Allyl-2-(3,4-dimethoxyphenyl)-3-methyl-2,3,$3_{\alpha}$,6-tetrahydro-6-oxabenzofuran

5-Allyl-2-(3,4-dimethoxyphenyl)-3-methyl-2,3-dihydro-6-oxabenzofuran (3.26 g, 10 mmole) is dissolved in 20 ml $CH_2Cl_2$ and 2.5 g of $Ag_2O$ (11 mmole) is added portion-wise. The mixture is stirred vigorously for 16 hours. The organic salt is filtered and solution concentrated to yield 5-allyl-2-(3,4-dimethoxyphenyl)-3-methyl-2,3,$3_{\alpha}$,6-tetrahydro-6-oxabenzofuran as a light yellow gum. This crude product was used directly in the next step without further purification.

Step E: Preparation of 5-Allyl-2-(3,4-dimethoxyphenyl)-3,$3_{\alpha,\alpha}$-dimethyl-2,3,$3_{\alpha}$,6-tetrahydrobenzofuran

In a 10 ml round bottom flask, 5-allyl-2-(3,4-dimethoxyphenyl)-3-methyl-2,3,$3_{\alpha}$,6-tetrahydro-6-oxabenzofuran (326 mg, 1 mmole) and 5 ml dry THF are stirred at -78°C under nitrogen atmosphere when n-BuLi (0.7 ml, 1.5M solution in THF/toluene) is introduced by a syringe. The mixture is stirred at -78°C for 30 minutes and temperature is gradually warmed up to -20°C. Methyl iodide (1 ml) is added and the mixture is allowed to warm up to ambient temperature and stirred for one hour. The reaction mixture is concentrated and flash columned (silica 10% ethyl acetate/hexane) to separate isomers. 5-Allyl-2-(3,4-dimethoxyphenyl)-3,$3_{\alpha,\alpha}$-dimethyl-2,3,$3_{\alpha}$,6-tetrahydrobenzofuran is obtained as an oil.

WHAT IS CLAIMED IS:

1. A compound of formula:

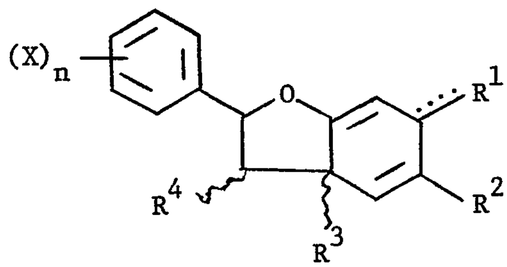

(I)

wherein

$R^1$ is (1) when it is joined to the ring by a double bond, O, S, $CR^5R^6$, or $NR^5$ where $R^5$ and $R^6$ independently represent H, loweralkyl, haloloweralkyl, loweralkenyl, aryl or aralkyl as defined below; or

(2) when it is joined to the ring by a single bond, $SR^5$, $SOR^5$, $SO_2R^5$, $NR^5R^6$, or $OR^7$ wherein $R^7$ represents $R^5$, $COR^5$, $COOR^5$, $CSR^5$ or $COSR^5$;

$R^2$ is (a) loweralkyl;

(b) loweralkenyl;

(c) loweralkoxy;

(d) lower alkynyl;

(e) aralkyl;

(f) haloloweralkyl;

$R^3$ is (a) loweralkyl;

(b) aryl;

(c) loweralkenyl;

(d) $OR^5$ where $R^5$ is as previously defined except that it cannot be $CH_3$;
(e) $SR^5$;
(f) $NR^5R^6$; or
(g) haloloweralkyl;

$R^4$ is
(a) loweralkyl;
(b) lower alkenyl;
(c) lower alkynyl;
(d) hydrogen; or
(e) aralkyl;

X is
(1) H;
(2) loweralkyl;
(3) loweralkenyl;
(4) loweralkynyl;
(5) aryl of 6 to 10 carbons;
(6) aralkyl;
(7) $R^5O-$;
(8) $R^5CO$;
(9) $R^5COO$;
(10) $R^5COS$;
(11) $R^5OCO$;
(12) $R^5SCO$;
(13) $R^5CONR^6$;
(14) $R^5NR^6CO$;
(15) $R^5R^6N$;
(16) $R^5S$;
(17) $R^5SO$;
(18) $R^5SO_2$;
(19) halo;
(20) $-O(CH_2)_mO-$ where m represents 1 or 2;
(21) $-(NH_2)C=NH$;

(22) -CN;

(23) $-NO_2$;

(24) $-(CH_2)_mOR^5$;

(25) $-(CH_2)_mCOOR^5$;

(26) heteroaryl or substituted heteroaryl; or

(27) halo loweralkyl; and

n is 1 to 5.

2. The compound of Claim 1 having the formula:

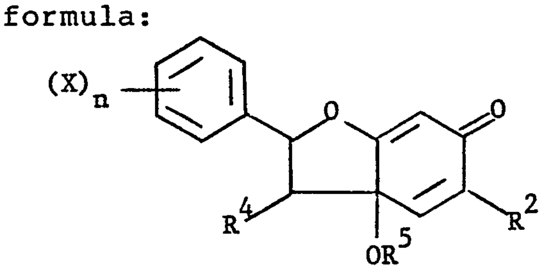

(II)

wherein $R^2$, $R^5$, $R^4$, X and n are as previously defined.

3. The compound of Claim 1 having formula:

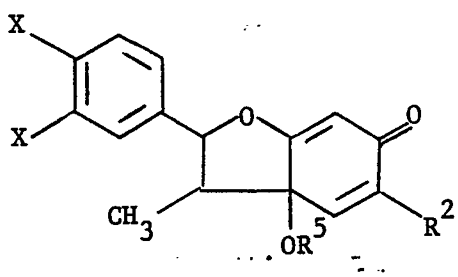

wherein $R^2$, $R^5$ and X are as previously defined.

4. The compound of Claim 1 having formula:

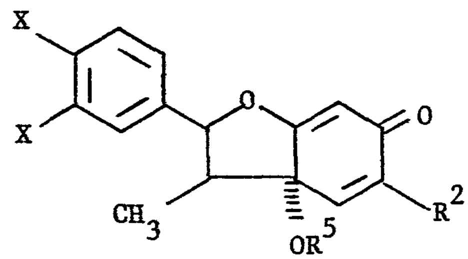

wherein $R^2$ and $R^3$ are as previously defined.

5. A process for preparing a compound of formula:

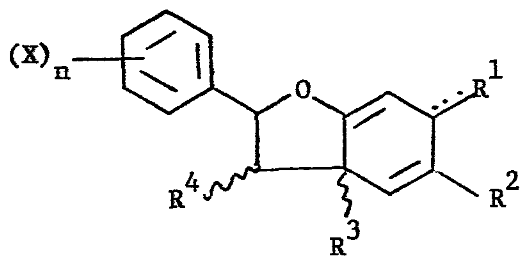

(I)

wherein

$R^1$ is (1) when it is joined to the ring by a double bond, O, S, $CR^5R^6$, or $NR^5$ where $R^5$ and $R^6$ independently represent H, loweralkyl, haloloweralkyl, loweralkenyl, aryl or aralkyl as defined below; or

(2) when it is joined to the ring by a single bond, $SR^5$, $SOR^5$, $SO_2R^5$, $NR^5R^6$, or $OR^7$ wherein $R^7$ represents $R^5$, $COR^5$, $COOR^5$, $CSR^5$ or $COSR^5$;

$R^2$ is
(a) loweralkyl;
(b) loweralkenyl;
(c) loweralkoxy;
(d) lower alkynyl;
(e) aralkyl;
(f) haloloweralkyl;

$R^3$ is
(a) loweralkyl;
(b) aryl;
(c) loweralkenyl;
(d) $OR^5$ where $R^5$ is as previously defined except that it cannot be $CH_3$;
(e) $SR^5$;
(f) $NR^5R^6$; or
(g) haloloweralkyl;

$R^4$ is
(a) loweralkyl;
(b) lower alkenyl;
(c) lower alkynyl;
(d) hydrogen; or
(e) aralkyl;

X is
(1) H;
(2) loweralkyl;
(3) loweralkenyl;
(4) loweralkynyl;
(5) aryl of 6 to 10 carbons;
(6) aralkyl;
(7) $R^5O-$;
(8) $R^5CO$;
(9) $R^5COO$;
(10) $R^5COS$;
(11) $R^5OCO$;
(12) $R^5SCO$;
(13) $R^5CONR^6$;

(14) $R^5NR^6CO$;
(15) $R^5R^6N$;
(16) $R^5S$;
(17) $R^5SO$;
(18) $R^5SO_2$;
(19) halo;
(20) $-O(CH_2)_mO-$ where m represents 1 or 2;
(21) $-(NH_2)C=NH$;
(22) -CN;
(23) $-NO_2$;
(24) $-(CH_2)_mOR^5$;
(25) $-(CH_2)_mCOOR^5$;
(26) heteroaryl or substituted heteroaryl; or
(27) halo loweralkyl; and

n is 1 to 5;

comprising:

(a) oxidizing a compound of formula (III):

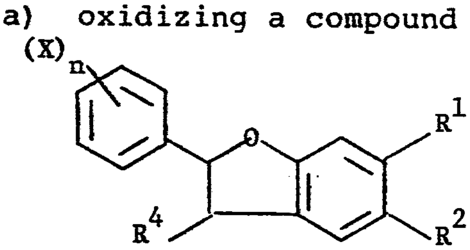

to a compound of formula IV:

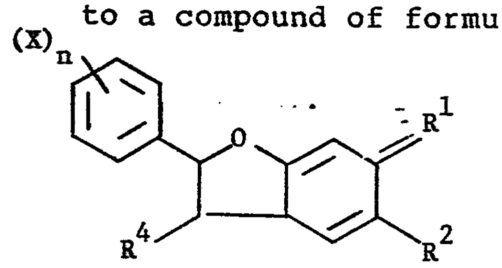

(b) treating the compound of formula IV with a reagent of formula $R^3L$ in the presence of a strong base wherein L is a good leaving group.

6. A pharmaceutical composition for treating PAF-induced conditions in mammalian species comprising a non-toxic pharmaceutical carrier and a therapeutically effective amount of a compound of formula:

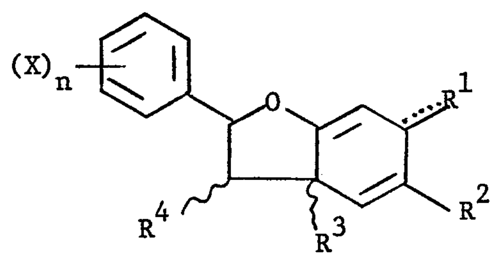

(I)

wherein

$R^1$ is (1) when it is joined to the ring by a double bond, O, S, $CR^5R^6$, or $NR^5$ where $R^5$ and $R^6$ independently represent H, loweralkyl, haloloweralkyl, loweralkenyl, aryl or aralkyl as defined below; or

(2) when it is joined to the ring by a single bond, $SR^5$, $SOR^5$, $SO_2R^5$, $NR^5R^6$, or $OR^7$ wherein $R^7$ represents $R^5$, $COR^5$, $COOR^5$, $CSR^5$ or $COSR^5$;

$R^2$ is (a) loweralkyl;

(b) loweralkenyl;

(c) loweralkoxy;

(d) lower alkynyl;

(e) aralkyl;

(f) haloloweralkyl;

$R^3$ is (a) loweralkyl;
(b) aryl;
(c) loweralkenyl;
(d) $OR^5$ where $R^5$ is as previously defined except that it cannot be $CH_3$;
(e) $SR^5$;
(f) $NR^5R^6$; or
(g) haloloweralkyl;

$R^4$ is (a) loweralkyl;
(b) lower alkenyl;
(c) lower alkynyl;
(d) hydrogen; or
(e) aralkyl;

X is (1) H;
(2) loweralkyl;
(3) loweralkenyl;
(4) loweralkynyl;
(5) aryl of 6 to 10 carbons;
(6) aralkyl;
(7) $R^5O-$;
(8) $R^5CO$;
(9) $R^5COO$;
(10) $R^5COS$;
(11) $R^5OCO$;
(12) $R^5SCO$;
(13) $R^5CONR^6$;
(14) $R^5NR^6CO$;
(15) $R^5R^6N$;
(16) $R^5S$;
(17) $R^5SO$;
(18) $R^5SO_2$;
(19) halo;

(20) $-O(CH_2)_mO-$ where m represents 1 or 2;

(21) $-(NH_2)C{=}NH$;

(22) $-CN$;

(23) $-NO_2$;

(24) $-(CH_2)_mOR^5$;

(25) $-(CH_2)_mCOOR^5$;

(26) heteroaryl or substituted heteroaryl; or

(27) halo loweralkyl; and

n is 1 to 5.

7. The pharmaceutical composition of Claim 6 wherein the compound is of formula:

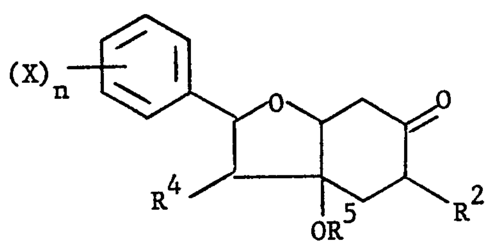

(II)

wherein $R^2$, $R^5$, $R^4$, X and n are as previously defined.

8. The pharmaceutical composition of Claim 6 wherein the compound is of formula:

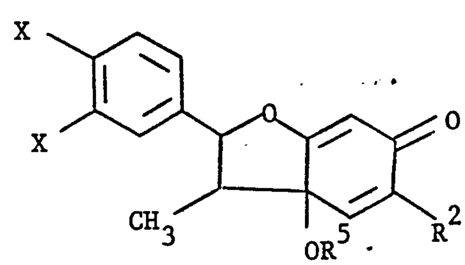

wherein $R^2$, $R^5$ and X are as previously defined.

9. The pharmaceutical composition of Claim 6 wherein the compound is of formula:

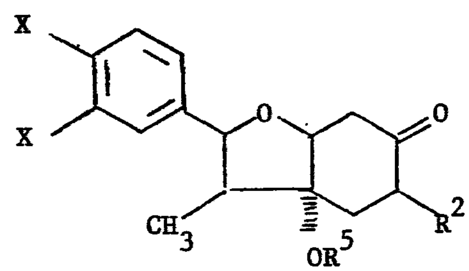

wherein $R^2$ and $R^3$ are as previously defined.

European Patent Office

**EUROPEAN SEARCH REPORT**

0159565

Application number

EP 85 10 3688

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 77, no. 15, no. 15, 9th October 1972, page 402, no. 101311x, Columbus, Ohio, US; O.A. LIMA et al.: "Chemistry of Brazilian Lauraceae. XX. Burchellin, a neoligna from Aniba burchellii" & PHYTOCHEMISTRY 1972, 11(6), 2031-2037<br>--- | 1-4 | C 07 D 307/80<br>A 61 K 31/34 //<br>C 07 C 43/23<br>C 07 C 39/19 |
| X | CHEMICAL ABSTRACTS, vol. 86, no. 3, 17th January 1977, page 193, no. 13793m, Columbus, Ohio, US; J.B. FERNANDES et al.: "The chemistry of Brazilian Lauraceae. Part 34. Neolignans from an Aniba species£ & PHYTOCHEMISTRY 1976, 15(6), 1033-1036<br>--- | 1-4 | TECHNICAL FIELDS SEARCHED (Int. Cl.4) |
| X | CHEMICAL ABSTRACTS, vol. 87, no. 19, 7th November 1977, page 565, no. 151909a, Columbus, Ohio, US; C.J. AIBA et al.: "The chemistry of Brazilian Lauraceae. Part 39. Benzofuranoid neolignans from Aniba simulans" & PHYTOCHEMISTRY 1977, 16(6), 741-743<br>--- | 1-4 | C 07 D 307/00 |
| X | CHEMICAL ABSTRACTS, vol. 93, no. 15, 13th October 1980, page 385, no. 146271f, Columbus, Ohio, US; R. BRAZ FILHO et al.: "The chemistry of Brazilian Lauraceae. Part 56. Neolignans from a Nectandra species" & PHYTOCHEMISTRY 1980, 19(4), 659-662<br>---/ | 1-4 | |

The present search report has been drawn up for all claims

| Place of search | Date of completion of the search | Examiner |
|---|---|---|
| THE HAGUE | 03-07-1985 | ALLARD M.S. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

0159565
Application number
EP 85 10 3688

Page 2

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| X | JOURNAL OF THE AMERICAN CHEMICAL SOCIETY, vol. 99, no. 24, 23rd November 1977, pages 8073-8075, Columbus, Ohio, US; G. BÜCHI et al.: "Biomimetic syntheses of the neolignans guianin, burchellin, 2-epi,3a-epiburchellin and futoenone" * Whole document * --- | 1-5 | |
| X | TETRAHEDRON LETTERS, vol. 24, no. 45, 1983, pages 5011-5012, Pergamon Press Ltd., Oxford, GB; Y. SHIZURI et al.: "Efficient synthesis of several aniba and magnolia neolignans" * Whole document * --- ----- | 1-5 | |

TECHNICAL FIELDS SEARCHED (Int. Cl.4)

The present search report has been drawn up for all claims

| Place of search | Date of completion of the search | Examiner |
|---|---|---|
| THE HAGUE | 03-07-1985 | ALLARD M.S. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document